# EUROPEAN PATENT APPLICATION

(11) **EP 4 810 582 A1**
(43) Date of publication of application: **23.09.2026**
(21) Application number: 24891448.3
(22) Date of filing: 14.11.2024
(51) Int. Cl.: C09K 5/10, F28D 15/02, H01L 23/36, H01L 23/373

(54) **HYDROFLUOROETHER COMPOSITION**

(30) Priority: 17.11.2023 JP 2023196146
(71) Applicant: AGC Inc., Chiyoda-ku Tokyo 100-8405 (JP)
(72) Inventor: SHIOTA, Hidefumi, Tokyo 100-8405 (JP); KAWAKAMI, Takafumi, Tokyo 100-8405 (JP); KAWAGUCHI, Satoshi, Tokyo 100-8405 (JP); TAKEUCHI, Yu, Tokyo 100-8405 (JP)
(74) Representative: Müller-Boré & Partner Patentanwälte PartG mbB
(86) International application number: PCT/JP2024/040394
(87) International publication number: WO 2025/105412

(57) **Abstract**

There is provided a hydrofluoroether composition capable of suppressing the formation of acid content even at high temperatures (for example, 90°C or higher). The hydrofluoroether composition comprises 1,1,1,2,3,3-hexafluoro-3-[2-(1,1,2,3,3,3-hexafluoropropoxy)ethoxy]propane, and 1,2,3,3,3-pentafluoro-1-[2-(1,1,2,3,3,3-hexafluoropropoxy)ethoxy]-1-propene, wherein the content of the 1,2,3,3,3-pentafluoro-1-[2-(1,1,2,3,3,3-hexafluoropropoxy)ethoxy]-1-propene is 0.0001% by mass or higher and lower than 2.0000% by mass.

## Description

### Technical Field

The present invention relates to a hydrofluoroether (HFE) composition useful for heat transfer media and the like.

### Background Art

The heat transfer media are used in various types of heat transfer means, such as temperature control of wafers in semiconductor production, cooling and heating of semiconductor devices and electronic parts, cooling of servers, and temperature control of heat pumps, heat pipes and thermostatic chambers.

Perfluorocarbon (PFC) has conventionally been widely used as the heat transfer media. PFC, however, has such problems as high global warming potentials (GWP) and high environmental impacts due to the greenhouse effect.

Hence, use of HFE having a lower environmental impact as an alternative heat transfer medium to PFC has been studied (see, for example, PTL1).

HFE, however, is lower in thermal stability than PFC; and when HFE is used continuously as a heat transfer medium, acid contents such as hydrogen fluoride are generated due to decomposition or elimination, leading to such risks as corrosion and deterioration, which in turn lead to breakdown of heat transfer medium circulation devices and the like.

As means to suppress decomposition of fluorine-based heat transfer media, there is known the addition of well-known stabilizers, for example, phenol compounds, unsaturated hydrocarbon group-containing aromatic compounds, aromatic amine compounds, aromatic thiazine compounds, terpene compounds, quinone compounds, nitro compounds, epoxy compounds or ortho ester compounds (see, for example, PTL2).

### Citation List

### Patent Literature

PTL1: Japanese Translation of PCT International Application Publication No. 2007-524737
PTL2: International Publication No. WO2016/181910

### Summary of Invention

### Technical Problem

However, for example, in the dry etching step using thermal plasma in the semiconductor production, in the case where an HFE heat transfer medium containing a known stabilizer added thereto is used under the severe conditions of a high electric field and a high-temperature environment (for example, 90°C or higher), the heat transfer medium may be partially decomposed, and therefore required characteristics (for example, high insulation) may not sufficiently be exhibited.

Hence, it is demanded that a heat transfer medium using HFE can suppress the formation of acid contents from the heat transfer medium over time without using much of a conventional stabilizer, and can be used stably and continuously as a heat transfer medium.

The present invention has been achieved in consideration of such a situation and has an object to provide an HFE composition capable of suppressing the formation of acid contents even at high temperatures of, for example, 90°C or higher.

### Solution to Problem

The present invention is based on such a finding that a combination of a predetermined HFE with a predetermined compound may suppress the formation of acid content even after the lapse of a few days at high temperatures of, for example, 90°C or higher.

The present invention provides the following means.
[1] A hydrofluoroether composition, comprising 1,1,1,2,3,3-hexafluoro-3-[2-(1,1,2,3,3,3-hexafluoropropoxy)ethoxy]propane and 1,2,3,3,3-pentafluoro-1-[2-(1,1,2,3,3,3-hexafluoropropoxy)ethoxy]-1-propene, wherein the content of the 1,2,3,3,3-pentafluoro-1-[2-(1,1,2,3,3,3-hexafluoropropoxy)ethoxy]-1-propene is 0.0001% by mass or higher and lower than 2.0000% by mass.
[2] The hydrofluoroether composition according to [1], wherein the content of the 1,2,3,3,3-pentafluoro-1-[2-(1,1,2,3,3,3-hexafluoropropoxy)ethoxy]-1-propene is 0.0001% by mass or higher and lower than 1.0000% by mass.
[3] A heat transfer medium, wherein the heat transfer medium is a hydrofluoroether composition according to [1] or [2].
[4] The heat transfer medium according to [3], wherein the heat transfer medium is used for cooling or heating a component member in a semiconductor production apparatus.

### Advantageous Effects of Invention

The HFE composition of the present invention can suppress the formation of acid content even at high temperatures of, for example, 90°C or higher. Hence, the HFE composition of the present invention can be used stably and continuously as a heat transfer medium.

### Description of Embodiments

An HFE composition of an embodiment (hereinafter, referred to also as the present embodiment) of the present invention comprises 1,1,1,2,3,3-hexafluoro-3-[2-(1,1,2,3,3,3-hexafluoropropoxy)ethoxy]propane (hereinafter, abbreviated to HFE-77-12) and 1,2,3,3,3-pentafluoro-1-[2-(1,1,2,3,3,3-hexafluoropropoxy)ethoxy]-1-propene, wherein the content of the 1,2,3,3,3-pentafluoro-1-[2-(1,1,2,3,3,3-hexafluoropropoxy)ethoxy]-1-propene is 0.0001% by mass or higher and lower than 2.0000% by mass.

HFE-77-12 is an HFE compound represented by chemical formula: CF₃CHFCF₂OCH₂CH₂OCF₂CHFCF₃. Since HFE-77-12 is liquid in a broad temperature range where while the boiling point is as high as 164°C, the freezing point is lower than - 100°C, HFE-77-12 can be used as an excellent heat transfer medium.

1,2,3,3,3-pentafluoro-1-[2-(1,1,2,3,3,3-hexafluoropropoxy)ethoxy]-1-propene is represented by chemical formula: CF₃CF=CFOCH₂CH₂OCF₂CHFCF₃, and is a compound having a structure in which hydrogen fluoride has been eliminated from a fluoroalkyl group bound to one etheric oxygen atom of HFE-77-12 (hereinafter, abbreviated to a dehydrofluorination compound).

The HFE composition of the present embodiment comprising HFE-77-12 and a minute amount of 0.0001% by mass or higher and lower than 2.0000% by mass (1 ppm by mass or higher and lower than 20,000 ppm by mass) of the dehydrofluorination compound can suppress the formation of acid content even after the lapse of a few days at high temperatures of 90°C or higher, even at 150°C.

The reason why the formation of acid content is suppressed is not clear, but it is presumed that even in the case where HFE-77-12 is decomposed at high temperatures to form acid content such as hydrogen fluoride, the acid content is captured by the minute amount of the dehydrofluorination compound in the HFE composition. When hydrogen fluoride is added to the dehydrofluorination compound, the dehydrofluorination compound is turned to HFE-77-12. Hence, even if the dehydrofluorination compound captures the acid content, no product having so greatly different structure as to greatly vary the characteristics as the heat transfer medium of the HFE composition is formed and the dehydrofluorination compound can conceivably act as a stabilizer for HFE-77-12.

Accordingly, in the case where the HFE composition of the present embodiment is used as a heat transfer medium, the corrosion of metal members and the like of heat transfer medium circulation devices by acid content originated from HFE can be suppressed.

The content of the dehydrofluorination compound in the HFE composition of the present embodiment is, from the viewpoint of satisfactorily suppressing the formation of acid content from the HFE composition, 0.0001% by mass or higher and lower than 2.0000% by mass (1 ppm by mass or higher and lower than 20,000 ppm by mass), and preferably 0.0001% by mass or higher and 1.0000% by mass or lower (1 ppm by mass or higher and 10,000 ppm by mass or lower) and more preferably 0.0010% by mass or higher and 0.1000% by mass or lower (10 ppm by mass or higher and 1,000 ppm by mass or lower).

Due to the fact that the content of the dehydrofluorination compound is 1 ppm by mass or higher, the formation of acid content from the HFE composition can be satisfactorily suppressed; but when the content is too high, it is not preferable because there arises a risk of hindering the characteristics of HFE-77-12 itself as a heat transfer medium. That is, when the content of the dehydrofluorination compound is 2.0000% by mass or higher, the formation of acid content from the HFE composition cannot sufficiently be suppressed. The reason therefor is not clear, but such a possibility is conceivable that when the dehydrofluorination compound is too much, acid content is formed due to decomposition and the like of the dehydrofluorination compound, exceeding the capturing power of acid content.

The content of HFE-77-12 in the HFE composition of the present embodiment is preferably 30% by mass or higher, more preferably 50% by mass or higher, still more preferably 70% by mass or higher and further still more preferably 80% by mass or higher.

In the case where the HFE composition of the present embodiment is used as a heat transfer medium, the content of HFE-77-12 itself in the HFE composition is, from the viewpoint of exhibiting favorable characteristics as a heat transfer medium of HFE-77-12 itself, preferably higher than 98.0000% by mass and 99.9999% by mass or lower, more preferably 99.0000% by mass or higher and 99.9990% by mass or lower and still more preferably 99.9000% by mass or higher and 99.9990% by mass or lower.

In the case where the HFE composition of the present embodiment is used as a heat transfer medium, the total content of HFE-77-12 and the dehydrofluorination compound is, from the viewpoint of stably exhibiting the characteristics as a heat transfer medium of HFE-77-12, preferably 99.0000% by mass or higher, more preferably 99.5000% by mass or higher and especially preferably 100% by mass.

The HFE composition of the present embodiment may be composed only of HFE-77-12 and the dehydrofluorination compound, but may contain components other than HFE-77-12 and the dehydrofluorination compound (for example, a fluorosolvent) in the range of not impairing the advantageous effects of the present invention according to various purposes including improvement of various characteristics.

In the case where the HFE composition of the present embodiment contains a fluorosolvent, the content of the fluorosolvent is preferably lower than 70% by mass, more preferably lower than 50% by mass, still more preferably lower than 30% by mass and further still more preferably lower than 20% by mass.

Examples of the fluorosolvent include commercially available products including products manufactured by Solvay S.A, such as Garden(R) HT90/110/135/150/170/200 (the aforementioned, CF₃-[OCF(CF₃)(CF₂)ₙ(OCF₂)ₘ]-CF₃), ZV90, SVX and ZT-180; products manufactured by Sanming Hexafluo Chemicals Co. Ltd. such as FTM-110/135/150/170/200/230/270; products manufactured by 3M Co. such as Fluorinert(R) FC-75 (C₈F₈)/3283 ((C₃F₇)₃N)/40 ((C₄F₉)₃N)/43 ((C₄F₉)₃N)/70 ((C₅F₁₁)₃N), FX-3300 (C₈F₈), Novec 7100 (mixture of CF₃(CF₂)₃OCH₃ and (CF₃)₂CFCF₂OCH₃)/7200 (mixture of (CF₃)(CF₂)₃OC₂H₅ and (CF₃)₂CFCF₂OC₂H₅)/7300 ((CF₃)₂CFCF(CF₂CF₃)OCH₃)/7500 (CF₃CF₂CF₂CF)(OCH₂CH₃)CF(CF₃)CF₃)/7600 (CF₃CFHCF₂OC(CH₃)CF₂CFHCF₃); products manufactured by Chemours Co. such as Opteon(R) SF10/30 (mixture of CF₃CH=CHCF₃ and CClH=CClH)/33 (CF₃CH=CHCF₃)/2P50; products manufactured by AGC Inc. such as Asahiklin(R) AC-2000 (CF₃CF₂CF₂CF₂CF₂CF₂H)/6000 (CF₃CF₂CF₂CF₂CF₂CF₂CH₂CH₃), and Amolea(R) AS-300 (CF₂HCF=CClH); products manufactured by Central Glass Co. Ltd. such as Cerefin(R) 1233Z (HCFO-1233zd(Z))/CGS-5E; products manufactured by Daikin Industries, LTD. such as DAISAVE(R) SS-54 ; and products manufactured by Grandit Co. Ltd. such as HFE-65-12.

The HFE composition of the present embodiment can be obtained by mixing HFE-77-12 and the dehydrofluorination compound. Methods for producing HFE-77-12 and the dehydrofluorination compound are not especially limited.

HFE-77-12 can be synthesized by a well-known method, for example, a method of addition reaction of hexafluoropropene with ethylene glycol in the presence of an alkali catalyst such as potassium carbonate.

The dehydrofluorination compound can be synthesized, for example, by a method of reacting HFE-77-12 in the presence of a strong base such as an alkali metal alkoxide to eliminate hydrogen fluoride.

Specifically, HFE-77-12 and the dehydrofluorination compound can be produced by methods described in Examples described below.

The HFE composition of the present embodiment, since being low in GWP, can suitably be used in broad applications, including detergents, solvents, foaming agents, aerosols, working media for heat pipes and for binary power generation for factory waste-heat recovery and the like, preservation solutions for electronic parts, and media for gross leak tests, thermal shock tests, liquid burn-in tests and withstand voltage tests for electronic parts.

Then, as described above, since the formation of acid content can be suppressed even at high temperatures of 90°C or higher, the HFE composition can suitably be used as heat transfer media.

Examples of the heat transfer media include those for use for temperature control of wafers in semiconductor production, cooling and heating of semiconductor devices and electronic parts, cooling of servers, and for heat pumps, heat pipes and thermostatic chambers.

The HFE composition of the present embodiment is suitable as a heat transfer medium for cooling, heating and the like of component members in semiconductor production apparatuses, and suitable also as a heat transfer medium to be used continuously under the severe conditions of a high electric field and a high-temperature environment, like in a dry etching step using thermal plasma in the semiconductor production.

Then, as described above, the HFE composition of the present embodiment can suitably be used also as a detergent. Specifically, the HFE composition can be used also for removal of stains accumulated in chambers of dry etching apparatuses used for semiconductor production (reaction products such as silicon oxide formed in etching steps).

### Examples

Hereinafter, the present invention will be described specifically based on Examples, but the present invention is not any more limited to the following Examples.

The identification of reaction products was carried out by using proton nuclear magnetic resonance spectra (¹H NMR), fluorine 19 nuclear magnetic resonance spectra (¹⁹F NMR) and gas chromatography mass spectrometry (GC-MS (used column: "DB-1301", length: 60 m, inner diameter: 250 µm, thickness: 1 µm, manufactured by Agilent Technologies, Inc.).

### [Synthesis of HFE-77-12]

In a stainless steel autoclave (internal volume: 2.1 L) equipped with a stirrer, 223 g of potassium carbonate (manufactured by Junsei Chemical Co. Ltd.), 200 g of ethylene glycol (manufactured by Junsei Chemical Co. Ltd.) and 400 g of an anhydrous acetonitrile (manufactured by Fujifilm Wako Pure Chemical Corp.) were charged and held hermetically at 20°C. The content in the autoclave was stirred and 966 g of hexafluoropropene (manufactured by AGC Inc.) in a gas state was added over 6 hours. The resultant was held at 20°C for 1 hour to be allowed to react, and thereafter, a reaction crude liquid in the autoclave was filtered and recovered.

The recovered reaction crude liquid contained 45% by mass of HFE-77-12 and 4.5% by mass of a dehydrofluorination compound.

Then, in a Hastelloy(R)-made autoclave (internal volume: 2.1 L), 1,000 g of the reaction crude liquid was charged, and 100 g of an anhydrous hydrogen fluoride (manufactured by AGC Inc.) was added and stirred for 1 hour. The obtained reaction crude liquid was phase separated and washed with water, and dried by Molecular Sieve 3A (manufactured by Fujifilm Wako Pure Chemical Corp.) and thereafter distillation purified to thereby obtain an HFE-77-12.

### [Synthesis of a dehydrofluorination compound]

In a glass-made reactor (internal volume: 0.5 L) equipped with a stirrer, 117 g of tert-butyl alcohol (manufactured by Kanto Chemical Co. Inc.) and 75 g of the HFE-77-12 were charged and heated at 40°C under stirring, and 100 g of potassium tert-butoxide (manufactured by Tokyo Chemical Industry Co. Ltd.) was added intermittently over 5 hours. The resultant was held at 40°C for 2 hours to be allowed to react, and thereafter, a reaction crude liquid was neutralized with a potassium hydrogencarbonate solution of 10% by mass in concentration and washed with water. An organic layer was recovered and distillation purified to thereby obtain a dehydrofluorination compound.

### [Examples 1 to 6] Preparation of HFE compositions

Compositions 1 to 6 were prepared by mixing the HFE-77-12 and the dehydrofluorination compound synthesized in the above so as to have respective proportions indicated in Examples 1 to 6 in Table 1.

### [High-temperature stability test]

Each HFE composition (the compositions 1 to 6) in Example 1 to 6 was evaluated by the following high-temperature stability test.

100 g of an HFE composition was put in a polytetrafluoroethylene (PTFE) container containing a test piece (25 mm × 30 mm, thickness: 2 mm) of a general cold rolled steel plate (SPCC) put therein to immerse the test piece, and stored at 100°C or 150°C for 7 days. The appearance of the test piece after the test was visually observed, and the high-temperature stability (presence/absence of the formation of acid content) of the HFE composition was evaluated according to the following evaluation criteria, and the evaluation result is shown in Table 1.

### <Evaluation criteria>

A: no change
B: disappearance of the surface gloss
C: rust formation on part of the surface
D: rust formation on the entire surface

In the cases of A and B of the evaluation, no rust formation on the test piece was observed and it was regarded that the formation of acid content from the HFE composition was suppressed. In the cases of C and D of the evaluation, rust formation on the test piece was observed and it was regarded that acid content was formed at high temperatures from the HFE composition.

**[Table 1]**

| Example | Composition No. | Content (Proportion in terms of mass) | | | High-temperature stability test | |
|---|---|---|---|---|---|---|
| | | HFE-77-12 | Dehydrofluorination compound | | 100°C | 150°C |
| 1 | 1 | 100.0000% | 0.0000% | (0ppm) | C | C |
| 2 | 2 | 99.9999% | 0.0001% | (1ppm) | B | B |
| 3 | 3 | 99.9990% | 0.0010% | (10ppm) | A | A |
| 4 | 4 | 99.9000% | 0.1000% | (1000ppm) | A | A |
| 5 | 5 | 99.0000% | 1.0000% | (10000ppm) | A | B |
| 6 | 6 | 98.0000% | 2.0000% | (20000ppm) | A | C |

From the results shown in Table 1, in the case (Example 1) where the HFE composition did not contain any dehydrofluorination compound and the content of the HFE-77-12 was 100% by mass, it is presumed that acid content was easily formed at the high temperatures of 100°C or higher; and in the HFE compositions (Examples 2 to 5) in which the content of the dehydrofluorination compound was 0.0001% by mass or higher and lower than 2.0000% by mass (1 ppm by mass or higher and lower than 20,000 ppm by mass), it is presumed that the formation of acid content including hydrogen fluoride was suppressed by the effect of capturing acid content of the dehydrofluorination compound.

In the case (Example 6) where the content of the dehydrofluorination compound in the HFE composition was 2.0000% by mass, it is presumed that due to that the dehydrofluorination compound was thermally decomposed at the high temperature of 150°C, and the decomposition exceeded the capturing power of acid content, the HFE composition formed acid content.

Then, also in the case where in a semiconductor production apparatus equipped with a mechanism of circulating a heat transfer medium and transferring heat, the HFE composition of Example 4 as a heat transfer medium was circulated at a control temperature of 100°C for 100 hours, use thereof with no problem was confirmed.

## Claims

1. A hydrofluoroether composition, comprising: 1,1,1,2,3,3-hexafluoro-3-[2-(1,1,2,3,3,3-hexafluoropropoxy)ethoxy]propane; and 1,2,3,3,3-pentafluoro-1-[2-(1,1,2,3,3,3-hexafluoropropoxy)ethoxy]-1-propene,
wherein a content of the 1,2,3,3,3-pentafluoro-1-[2-(1,1,2,3,3,3-hexafluoropropoxy)ethoxy]-1-propene is 0.0001% by mass or higher and lower than 2.0000% by mass.

2. The hydrofluoroether composition according to claim 1, wherein the content of the 1,2,3,3,3-pentafluoro-1-[2-(1,1,2,3,3,3-hexafluoropropoxy)ethoxy]-1-propene is 0.0001% by mass or higher and lower than 1.0000% by mass.

3. A heat transfer medium, wherein the heating medium is a hydrofluoroether composition according to claim 1 or 2.

4. The heat transfer medium according to claim 3, wherein the heat transfer medium is used for cooling or heating a component member in a semiconductor production apparatus.
